Europäisches Patentamt

European Patent Office

Office européen des brevets

(19)

(11) EP 0 399 605 B1

# (12) EUROPEAN PATENT SPECIFICATION

(45) Date of publication and mention
of the grant of the patent:
**14.02.1996 Bulletin 1996/07**

(51) Int Cl.$^6$: **C07K 1/00**, C07K 5/075

(21) Application number: **90201273.1**

(22) Date of filing: **19.05.1990**

(54) **Method for crystallizing alpha-L-aspartyl-L-phenylalanine methyl ester**

Verfahren zur Kristallisierung von alpha-Asparaginsäure-L-phenylalaninmethylester

Méthode pour cristalliser l'ester méthylique de l'alpha-aspartyl-L-phenylalanine

(84) Designated Contracting States:
**AT BE CH DE DK ES FR GB IT LI NL SE**

(30) Priority: **22.05.1989 JP 126696/89**

(43) Date of publication of application:
**28.11.1990 Bulletin 1990/48**

(73) Proprietors:
- **STAMICARBON B.V.**
  **NL-6167 AC Geleen (NL)**
- **TOSOH CORPORATION**
  **Yamaguchi 746 (JP)**

(72) Inventors:
- **Irino, Shigeaki**
  **NL-6241 EV Bunde (NL)**

- **Wakamatsu, Hidetoshi**
  **Shin-Nanyo-shi, Yamaguchi-ken (JP)**
- **Kunisawa, Yukio**
  **Shin-Nanyo-shi, Yamaguchi-Ken (JP)**
- **Stoelwinder, Christiaan Johannes Cornelis**
  **NL-6137 HC Sittard (NL)**
- **Toussaint, Antoon Gerard Theodoor**
  **NL-6416 GX Heerlen (NL)**

(74) Representative:
**Hoogstraten, Willem Cornelis Roeland et al**
**NL-6160 MA Geleen (NL)**

(56) References cited:
**EP-A- 0 128 694**

Remarks:
The file contains technical information submitted
after the application was filed and not included in
this specification

## Description

The present invention relates to a method for crystallizing and separating $\alpha$-L-aspartyl-L-phenylalanine methyl ester (hereinafter referred to simply as APM).

APM is a useful peptide type sweetener having a sweetness of about 200 times that of sugar. APM can be synthesized by various methods. In any method, it is finally crystallized from a hot aqueous solution by cooling after a purification step, and the crystals are separated by a solid-liquid separation apparatus such as a centrifugal separator, then dehydrated and dried to obtain a final product. Such crystallization by cooling is usually conducted in a stirred crystallizer having a heat conductive surface for cooling or in a crystallizer equipped with a heat exchanger in an external circulation system. However, it may be conducted also by a crystallizer designed for the purpose of improving the crystal properties wherein cooling is conducted solely by heat conduction so as to avoid a forced flow (EP-B-0.091.787).

When subjected to crystallization under cooling by a crystallizer involving usual stirring or a forced flow such as external circulation, APM usually gives fine crystals which are poor in the filtration and dehydration efficiency. Further, in such a method, crystals readily precipitate on the heat conducting surface for cooling, thus leading to so-called scaling, whereby the heat conduction efficiency rapidly decreases, and it used to be necessary to stop the crystallization operation frequently to remove scales.

To avoid such a problem, a method has been proposed wherein an APM aqueous solution is cooled by heat conduction without imparting a forced flow such as mechanical stirring, to form a pseudo-solid phase, and thereafter further cooled, as the case requires (EP-B-0.091.787). According to this method, it is possible to obtain crystals having improved filtration and dehydration properties in the solid-liquid separation step. However, cooling efficiency is extremely poor, since the cooling is conducted by heat conduction without stirring and yet such cooling is continued even after formation of the pseudo-solid phase. This is extremely disadvantageous in the case of crystallizing a substance such as APM which is readily decomposed by heat to give 5-benzyl-3,6-dioxo-2-piperazine acetic acid having no sweetness.

Accordingly, in this method, it is impossible to use a commonly industrially employed apparatus such as a tank type crystallizer, and it is necessary to employ a special crystallizer having a large heat conducting surface and capable of withdrawing the pseudo-solid phase, as proposed in EP-B-0.091.787. Such a crystallizer is naturally poor in the cooling efficiency, the apparatus is expensive, and thus the method is not suitable as an industrial method for crystallization.

The present inventors have conducted extensive studies to solve the above-mentioned problems involved in the crystallization of APM. As a result, they have found an industrially advantageous method for crystallizing APM, which provides crystals excellent in the filtration and dehydration properties.

Namely, it has been surprisingly found that when a hot aqueous solution containing APM (hereinafter referred to simply as solution A) and a cold aqueous solution containing APM crystals (hereinafter referred to simply as solution B) are mixed in such proportions that the APM concentration after mixing exceeds the solubility of APM in the mixture at the temperature of the mixture, thus to break the supersaturation to crystallize APM, it is possible to obtain crystals excellent in the filtration and dehydration properties even under a forced flow condition such as mechanical stirring, and it is possible to conduct crystallization without any substantial scaling on the wall surface or the heat conducting surface for cooling of the crystallizer during such operation of breaking the supersaturation or during the subsequent cooling operation which is conducted as the case requires.

Each of the aqueous solutions A and B, or both of them, also may contain a lower alcohol such as methanol, ethanol or propanol. Use of such lower alcohol containing solutions has the advantage that higher yields of crystals can be obtained.

Such advantage is being taught in EP-A-0.128.694 which is aimed at high productivity crystallization of APM by making use of the fact that APM solubility at elevated temperatures may be two to four or more times as large as that in for instance methanol or water alone at the same temperature, whereas such differences are much smaller at low temperature. Crystallization according to EP-A-0.128.694 may be effected by concentrating the solution, and/or by cooling the solution (directly or indirectly), and/or by addition of water and/or a lower alcohol to the solution to change its composition. However EP-A-0.128.694 nowhere suggests that such addition of pure cold solvent is preferable: on the contrary, it is shown that this results in the highest level of impurities in the crystals obtained. Therefore there is no suggestion for using cold APM containing aqueous solutions, particularly not for using cold APM solutions which contain APM crystals.

In the present invention the amount of lower alcohol in each of the solutions A and B may vary over a wide range. Especially, the amount of lower alcohol in solution B may be very high. By using such lower alcohol containing aqueous solutions for solution B very low temperatures of this solution B may be used at the start of the mixing step, thus giving quick and effective cooling and yielding crystals with excellent filtration and dehydration properties. All such embodiments are thought to be encompassed by the term aqueous solution.

This is certainly a surprising new fact in view of the peculiar crystallization behavior of APM because of which it has been believed that the crystal properties can not be improved by any means under a forced flow condition such as stirring (Kishimoto and Naruse, 'Chemistry and Industry', February 16, 1987 issue, p. 127-128; 'Journal of Chemical Technology and Biotechnology', Vol. 43, p. 71-82, 1988).

Thus, the present invention provides a method for cyrstallizing $\alpha$-L-aspartyl-L-phenylalanine methyl ester, which comprises mixing a hot aqueous solution containing a-L-aspartyl-L-phenylalanine methyl ester and a cold aqueous solution containing $\alpha$-L-aspartyl-L-phenylalanine methyl ester crystals, each of which aqueous solutions may contain methanol, ethanol or propanol, in such proportions that the concentration of a-L-aspartyl-L-phenylalanine methyl ester after mixing exceeds its solubility in the mixture of the two aqueous solutions at the temperature of the mixture, to precipitate crystals of a-L-aspartyl-L-phenylalanine methyl ester, and if necessary, further cooling the mixture, for obtaining a higher yield of crystals.

Now, the present invention will be described in detail with reference to the preferred embodiments.

The APM content in solution A being one of the solutions used in the present invention, is set at a level higher than the solubility of APM at the temperature of the mixture, when solution A is mixed with solution B being the other solution. However, from the viewpoint of the yield of APM crystals from the crystallization, the APM content in solution A is preferably usually at least 15 g/l. The upper limit of the APM content may exceed the solubility at the temperature of the hot aqueous solution to be mixed, but it is usually not higher than the solubility. The temperature of the hot aqueous solution is usually within a range of from 30 to 100°C. In view of the fact that APM is readily decomposed at a high temperature, it is preferably from 30 to 80°C.

The primary purpose of solution B to be used as the other solution in the present invention is to reduce, when mixed with solution A, the temperature of solution A without relying on heat conduction and to create a supersaturated state of APM in the mixture, so that by the subsequent operation for breaking the supersaturation, APM is precipitated as crystals having excellent filtration and dehydration properties.

The cold aqueous solution contains APM crystals in the form of a slurry. The temperature of solution B may be at any level so long as it is lower than the temperature of solution A, but it is usually within a range of from 0 to 25°C, and may even be much lower if solution B is an aqueous solution containing a lower alcohol.

In the method of the present invention, solutions A and B may be mixed in optional proportions. In such a case, however, the proportions are, of course, set at optional levels within such a range that the APM concentration after mixing exceeds the solubility at the temperature of the mixture of the two solutions.

The mixing of the two solutions is conducted to such an extent that the two solutions are sufficiently mixed. For example, the mixing can be conducted by means of a usual industrially useful mixing apparatus such as a mixing tank with a stirrer or an in-line mixer. The mixing may be conducted by introducing the two solutions simultaneously to the mixing apparatus. Otherwise, one of the solutions may be introduced first, and the other solution is then introduced and mixed with the first solution. When the two solutions are introduced simultaneously, such introduction may be conducted by a batch system, or the introduction into and the withdrawal from the mixing apparatus may be conducted continuously.

When the two solutions are mixed, a supersaturated state of APM is created, and consequently APM precipitates as crystals. The time required till the precipitation depends on the degree of supersaturation of APM. Namely, if the degree of supersaturation is low, the time is long, and if the degree of supersaturation is high, crystallization starts immediately after the mixing. In a case where APM crystals are present in a large amount in solution B, and the degree of supersaturation of APM after the mixing is high, larger crystals in solution B remain crystalline, while fine crystals of APM in solution B will dissolve completely, and the larger crystals will be co-existent with crystals precipitated afresh.

The crystals thus obtained are usually superior in the filtration and dehydration properties to APM crystals in solution B used for the mixing. In a case where the amount of APM crystals in solution B is small, and the degree of supersaturation of APM after mixing is low, APM crystals will be dissolved to form a uniform supersaturated solution, and thereafter APM crystals start to precipitate.

After mixing the two solutions, APM crystals precipitated by breaking of the supersaturation, may be isolated by solid-liquid separation. In order to maximize the yield, it is of course advisable to wait until the degree of supersaturation adequately diminishes before conducting the operation for solid-liquid separation. When a higher yield of crystals is desired, the solution mixture may further be cooled to attain the object.

The operation to break the supersaturated state may be conducted under a forced flow state by e.g. stirring or external circulation, or may be conducted without imparting such a forced flow. However, in a case where the operation to break the supersaturated state involves a cooling operation, crystallization can be conducted at a higher cooling efficiency under a forced flow condition as compared with the case where crystallization is conducted without giving a forced flow.

APM crystals thus precipitated can be recovered by a conventional method.

According to the present invention, it is possible to obtain crystals having excellent filtration and dehydration properties, and thus the present invention provides an industrial process which is economically advantageous particularly in the step of crystallization and subsequent steps. Namely, in the solid-liquid separation step, the installation can be simplified due to the improvement of the filtration properties. Further, efficiency for washing impurities in the attached mother liquor, such as DKP, is remarkably improved. Furthermore, due to the improvement of the dehydration properties, the drying load in the drying step is reduced.

**EP 0 399 605 B1**

Further, according to the present invention, the hot aqueous solution is immediately cooled when mixed with the cold aqueous solution, whereby decomposition of APM is substantially reduced, and thus product of high purity is obtained.

Furthermore, the cooling efficiency is remarkably improved as compared with cooling by heat conduction without a forced flow.

According to the present invention, no substantial scaling forms in the crystallizer, whereby frequency for a cumbersome operation for the removal of scales can remarkably be reduced.

As is apparent from the foregoing, the present invention provides an industrially extremely useful method for crystallization which gives crystals having excellent filtration and dehydration properties by overcoming various drawbacks inherent to the conventional crystallization operations including crystallization by cooling under forced flow and crystallization involving the formation of a pseudo-solid phase without a forced flow.

Now, the present invention will be described in further detail with reference to Examples. However, it should be understood that the present invention is by no means restricted to such specific Examples.

In the following examples, the specific resistance of the filter cake was calculated based upon the filtration rate, which was determined in accordance with the following method.

500 g of a slurry containing formed crystals was sampled, and filtration was conducted at a differential pressure of 400 mm Hg ($\Delta P = 53329$ Pa) while pouring the slurry to a suction filter (leaf tester) provided with a polypropylene filter cloth with an air permeation of 5 $ml/cm^2.sec$ (12 mm $H_2O$), i.e. having a resistance of $1.3 \times 10^8$ 1/m, so that the crystals were maintained on the filter cloth. The filtration rate was calculated based on the time from the initiation of the filtration till no longer slurry remained on the filter cloth after the entire amount was poured and the amount of the filtrate at the time. Two calculations were used:

a) a quick estimate (for rough comparison purposes) by dividing the volume of the filtrate by the measured time (in seconds) and multiplying this figure by 60 and the reciprocal of the filter area;

b) a calculation based on theoretical considerations, according to a formula originated from Poiseuille and d'Arcy:

$$\frac{t}{V} = \frac{\alpha \cdot \eta \cdot C}{2\Delta P.A^2} \cdot V + \frac{R_m \cdot \eta}{\Delta P.A} \qquad (s/m^3)$$

wherein

$t =$     filtration time (s)
$V =$     volume of filtrate ($m^3$)
$\alpha =$     specific resistance of filter cake (m/kg)
$\eta =$     dynamic viscosity of filtrate (Pa.s)
$\Delta P =$     differential pressure across filter (Pa)
$C =$     mass of crystals deposited per unit volume of filtrate obtained ($kg/m^3$)
$R_m =$     resistance of the filter cloth (1/m)
$A =$     filtration area ($m^2$)

[ here: $\Delta P = 53229$ Pa, Rm = $1.3 \times 10^8$ 1/m,
      A = 1/127 $m^2$]

The figures according to calculation b) are used for determination of the specific resistance, as presented in the summarizing table below. This table also provides the figures of filtrate amounts and time.

<u>COMPARATIVE EXAMPLE</u>

Into a glass container (125 mm in inner diameter) equipped with an external cooling jacket and a stirrer (paddle type, 108 mm in diameter) and having an internal capacity of 2,500 ml, 2,000 ml of an aqueous APM solution of 60°C containing APM at a concentration of 3.5% by weight was poured, the temperature in the crystallizer was lowered at a rate of 5°C per 10 minutes by circulating a cooling medium to the jacket from an external water bath while stirring the solution at a rotational speed of 200 rpm. After the temperature in the crystallizer reached 5°C, stirring was continued at the same temperature at a rotational speed of 200 rpm for about 10 minutes. The filtration rate of this slurry was 48 $1/m^2.min$ as measured by leaf tester (355 ml filtrate in 56.4 sec.). Substantial scaling was observed at the cooling surface of the crystallizer.

EXAMPLE 1

Into the same crystallizer as used in Comparative Example 1, 1,000 ml of the aqueous APM slurry of 5°C obtained in Comparative Example 1 containing APM at a concentration of 3.5% by weight, was charged, and then 1,000 ml of an aqueous APM solution of 60°C containing APM at a concentration of 3.5% by weight, was added thereto, while stirring the slurry at a rotational speed of 200 rpm. The temperature of the mixture at that time was 32.5°C. Stirring was continued for 10 minutes at a rotational speed of 200 rpm, and the temperature in the crystallizer was lowered at a rate of 5°C per 10 minutes by circulating a cooling medium to the jacket of the container while gradually lowering the temperature of the external water bath. After the temperature in the crystallizer reached 5°C, stirring was continued at the same temperature at a rotational speed of 200 rpm for about 10 minutes. The filtration rate of this slurry was 134 $1/m^2$.min as measured by leaf tester (360 ml filtrate in 20.5 sec.). Further, no substantial scaling was observed at the cooling surface of the crystallizer.

EXAMPLE 2

Into the same crystallizer as used in Comparative Example 1, 800 ml of an aqueous APM slurry of 5°C obtained in Comparative Example 1 containing APM at a concentration of 3.5% by weight was charged. Then, 1,200 ml of an aqueous APM solution of 50°C containing APM at a concentration of 3.5%, was added thereto while stirring the slurry solution at a rotational speed of 200 rpm. Stirring of the solution was continued for 10 minutes at a rotational speed of 200 rpm without circulating a cooling medium to the jacket of the container from an external water bath. The filtration rate of this slurry was 338 $1/m^2$.min as measured by leaf tester (342 ml filtrate in 7.7 sec.). Further, no substantial scaling was observed at the cooling surface of the crystallizer.

EXAMPLE 3

600 ml of an aqueous APM solution of 50°C containing APM at a concentration of 3.5% by weight, was put into a beaker. While stirring the solution by a magnetic stirrer, 400 ml of an aqueous APM slurry of 5°C obtained in Comparative Example 1 containing APM at a concentration of 3.5% by weight, was added and mixed thereto. The temperature of the mixture at that time was 32°C. This mixture was poured into a glass cylindrical container equipped with an external cooling jacket and a rubber stopper at the bottom and having an inner diameter of 50 mm and a height of 600 mm. The mixture was left to stand for 10 minutes, and then a cooling medium cooled to 5°C by an external water bath was circulated in the jacket.

One hour later, the rubber stopper at the bottom of the container was removed, and the content was withdrawn into a beaker and stirred with a motor-driven mechanical stirrer at a rotational speed of 200 rpm for 5 minutes. The temperature of the slurry at that time was 5°C, and the filtration rate was 109 $l/m^2$.min as measured by leaf tester (365 ml filtrate in 25.5 sec.). The crystals were readily withdrawn from the cylindrical container, and no substantial scaling was observed at the wall surface.

These Examples and the Comparative Example may be summarized as follows, using the calculations as indicated above:

| Example | leaf tester filtrate (ml) | time (sec.) | filtration rate $(l/m^2.min.)$ *) | **) | specific resistance of filter cake $(m/kg)$**) |
|---|---|---|---|---|---|
| Comp. | 355 | 56.4 | 48 | 47 | $6.00 \times 10^{10}$ |
| 1 | 360 | 20.5 | 134 | 78 | $2.11 \times 10^{10}$ |
| 2 | 342 | 7.7 | 338 | 122 | $2.59 \times 10^{10}$ |
| 3 | 365 | 25.5 | 109 | 71 | $2.56 \times 10^{10}$ |

*) According to calculation method a)
**) According to calculation method b)

## Claims

1. A method for crystallizing α-L-aspartyl-L-phenylalanine methyl ester, which comprises mixing a hot aqueous solution containing α-L-aspartyl-L-phenylalanine methyl ester and a cold aqueous solution containing α-L-aspartyl-L-phenylalanine methyl ester crystals, each of which aqueous solutions may contain methanol, ethanol or propanol, in such proportions that the concentration of α-L-aspartyl-L-phenylalanine methyl ester after mixing exceeds its solubility in the mixture of the two aqueous solutions at the temperature of the mixture, to precipitate crystals of α-L-aspartyl-L-phenylalanine methyl ester, and when a higher yield of crystals is desired the solution mixture may be further cooled.

2. The method according to claim 1, wherein the content of a-L-aspartyl-L-phenylalanine methyl ester in the hot aqueous solution is at least 15 g/l.

3. The method according to claim 1 or 2, wherein the content of α-L-aspartyl-L-phenylalanine methyl ester in the hot aqueous solution does not exceed its solubility in the hot aqueous solution.

4. The method according to one of claims 1-3, wherein the temperature of the hot aqueous solution is within a range of from 30 to 100°C.

5. The method according to one of claims 1-4, wherein one or both of the hot and cold aqueous solutions also contain(s) methanol, ethanol or propanol.

6. The method according to one of claims 1-5, wherein the temperature of the cold aqueous solution is within a range of from 0 to 25°C.

## Patentansprüche

1. Verfahren zur Kristallisation von a-L-Aspartyl-L-phenylalaninmethylester, welches umfaßt: Mischen einer heißen wässerigen Lösung, enthaltend a-L-Aspartyl-L-phenylalaninmethylester, und einer kalten wässerigen Lösung, enthaltend α-L-Aspartyl-L-phenylalaninmethylester-Kristalle, wobei jede der wässerigen Lösungen Methanol, Ethanol oder Propanol enthalten kann, in derartigen Mengen, daß die Konzentration von α-L-Aspartyl-L-phenylalaninmethylester nach dem Mischen seine Löslichkeit in der Mischung der beiden wässerigen Lösungen bei der Temperatur der Mischung überschreitet, wobei Kristalle von a-L-Aspartyl-L-phenylalaninmethylester ausgefällt werden, und, wenn eine höhere Ausbeute an Kristallen gewünscht wird, weiteres Abkühlen der Lösungsmischung.

**2.** Verfahren nach Anspruch 1, bei welchem der Gehalt an $\alpha$-L-Aspartyl-L-phenylalaninmethylester in der heißen wässerigen Lösung zumindest 15 g/l beträgt.

**3.** Verfahren nach Anspruch 1 oder 2, bei welchem der Gehalt an a-L-Aspartyl-L-phenylalaninmethylester in der heißen wässerigen Lösung seine Löslichkeit in der heißen wässerigen Lösung nicht überschreitet.

**4.** Verfahren nach einem der Ansprüche 1 bis 3, bei welchem die Temperatur der heißen wässerigen Lösung im Bereich von 30 bis 100°C liegt.

**5.** Verfahren nach einem der Ansprüche 1 bis 4, bei welchem eine oder beide der heißen und kalten wässerigen Lösungen auch Methanol, Ethanol oder Propanol enthält bzw. enthalten.

**6.** Verfahren nach einem der Ansprüche 1 bis 5, bei welchem die Temperatur der kalten wässerigen Lösung im Bereich von 0 bis 25°C liegt.

**Revendications**

**1.** Une méthode pour cristalliser de l'ester méthylique de l'alpha-aspartyl-L-phenylalanine, comprenant les étapes consistant à mélanger une solution aqueuse chaude contenant de l'ester méthylique de l'alpha-aspartyl-L-phenylalanine et une solution aqueuse froide contenant des cristaux d'ester méthylique de l'alpha-aspartyl-L-phenylalanine, chacune des solutions aqueuses pouvant contenir du méthanol, de l'éthanol ou du propanol, dans des proportions telles que la concentration de l'ester méthylique de l'alpha-aspartyl-L-phenylalanine après mélange excède son degré de solubilité dans le mélange des deux solutions aqueuses à la température du mélange, afin de précipiter des cristaux d'ester méthylique de l'alpha-aspartyl-L-phenylalanine, et, au besoin, consistant à prolonger le refroidissement du mélange de la solution afin d'obtenir un rendement plus élevé de cristaux.

**2.** La méthode selon la Revendication 1, dans laquelle la teneur en ester méthylique de l'alpha-aspartyl-L-phenylalanine dans la solution aqueuse chaude est au moins égale à 15 g/l.

**3.** La méthode selon la Revendication 1 ou 2, dans laquelle la teneur en ester méthylique de l'alpha-aspartyl-L-phenylalanine dans la solution aqueuse chaude n'excède pas sa solubilité dans la solution aqueuse chaude.

**4.** La méthode selon l'une des Revendications 1 à 3, dans laquelle la température de la solution aqueuse chaude est comprise dans la gamme allant de 30 à 100°C.

**5.** La méthode selon l'une des Revendications 1 à 4, dans laquelle l'une ou les deux solutions aqueuses chaude et froide contien(nen)t en outre du méthanol, de l'éthanol ou du propanol.

**6.** La méthode selon l'une des Revendications 1 à 5, dans laquelle la température de la solution aqueuse froide est comprise dans la gamme allant de 0 à 25°C.